(19) Europäisches Patentamt
European Patent Office
Office européen des brevets

(11) EP 0 976 328 B1

(12) **EUROPEAN PATENT SPECIFICATION**

(45) Date of publication and mention of the grant of the patent:
**06.10.2004 Bulletin 2004/41**

(21) Application number: **99114117.7**

(22) Date of filing: **19.07.1999**

(51) Int Cl.[7]: **A01N 51/00**, A01N 47/40, A01N 43/88, A01N 43/78, A01N 43/64, A01N 43/54, A01N 43/50, A01N 43/40, A01N 43/08, A01N 37/52, A01N 37/34, A01N 25/02

(54) **Ectoparasite controlling agent for animals**

Mittel zur Bekämpfung von Ektoparasiten bei Tieren

Agent pour lutter contre les ectoparasites sur des animaux

(84) Designated Contracting States:
**DE ES FR GB IT**

(30) Priority: **27.07.1998 JP 21087598**
**28.10.1998 JP 30696098**

(43) Date of publication of application:
**02.02.2000 Bulletin 2000/05**

(73) Proprietor: **Sumitomo Chemical Company, Limited**
**Chuo-ku Osaka 541-8550 (JP)**

(72) Inventors:
- **Sembo, Satoshi**
  **Takarazuka-shi, Hyogo 665-0043 (JP)**
- **Makita, Mitsuyasu**
  **Nishinomiya-shi, Hyogo 663-8001 (JP)**

(74) Representative: **VOSSIUS & PARTNER**
**Siebertstrasse 4**
**81675 München (DE)**

(56) References cited:
**EP-A- 0 061 208**
**EP-A- 0 682 869**
**WO-A-97/37544**
**WO-A-97/40692**
**WO-A-98/17277**
**WO-A-98/42191**
**WO-A-99/41987**

- **ARTHER R ET AL: "Efficacy of imidacloprid for removal and control of fleas (Ctenocephalides felis) in dogs" AMERICAN JOURNAL OF VETERINARY RESEARCH, vol. 58, no. 8, August 1997 (1997-08), pages 848-850, XP002115500 ISSN: 0002-9645**

EP 0 976 328 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to ectoparasite controlling agents, and more specifically relates to an excellent ectoparasite controlling agent for animals which is used for regional application methods such as spot-on or pour-on application and a method for controlling ectoparasites of animals.

Heretofore, as an application method to control harmful pests that injure domestic animals such as pets and farm animals, particularly ectoparasites such as fleas (Pulicidae) and lice (Anoplura), methods of application recognized as spot-on and pour-on applications have been known, and because these methods of applications have been elementary, they are utilized in the concerning ectoparasite controlling.

WO-A-98/17277 discloses compounds and compositions for killing both ecto and endo parasites which comprises administering to a warm blooded animal at least a systemically effective amount of a defined amidine or its salt.

In WO-A-99/41987 water-containing formulations for the dermal control of parasitic insects and mites on humans are described. The formulations of WO-A-99/41987 have the following composition: a) agonists or antagonists of the nicotinic acetylcholine receptors of insects in a concentration of from 0.0001 to 7.5% by weight; b) water in a concentration of from 20 to 50% by weight; c) acyclic alcohols in a concentration of from 20 to 50% by weight; d) solvents from the group consisting of cyclic carbonates and lactones in a concentration of from 2.5 up to 20.0% by weight; and e) optionally further auxiliaries from the group consisting of thickeners, spreading agents, colorants, antioxidants, propellants, preservatives, tackifiers, emulsifiers, in a concentration of from 0 up to 30% by weight, each based on the overall weight of the formulation.

EP-A-682 869 relates to controlling of parasitic insects on humans and animals, e.g., fleas, lice and flies, by the non-systemic use of an agonist or antagonist of the insect's nicotinergic acetylcholine receptors.

Furthermore, EP-A-61 208 discloses a pour-on or spot-on method of eradicating or controlling ectoparasites and myiasis in non-human animals by the direct application of an ectoparasiticidal amount of a special pyrethroid as defined to a specific skin area of the non-human animal, as well as compositions for use in such a method and a process of preparing such compositions.

However, it was not common to have the active ingredients contained in the ectoparasite controlling agents to be always sufficiently effective.

The present invention provides an ectoparasite controlling agent for animals having an ability to exert an excellent efficacy against ectoparasites from spot-on or pour-on applications, with the incorporation of a neonicotinoid compound and a glycol or a specific glycol derivative, as well as from the specialized mixing ratio of that incorporation.

More specifically, the present invention is an ectoparasite controlling agent for animals (hereinafter, the present agent(s)) which is a liquid formulation comprising 10 to 95% by weight of a glycol or glycol monoalkyl ether (e. g. $C_{1-4}$ alkyl ether) and 0.1 to 20% by weight of the neonicotinoid compound given in the following formula (1) (herein after, the present compound(s)):

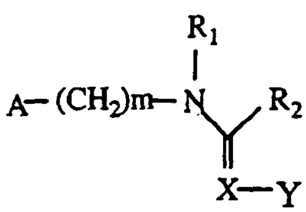

(1)

wherein, A represents a tetrahydrofuran-2-yl, tetrahydrofuran-3-yl, or 5-methyltetrahydrofuran-3-yl group;
$R_1$ represents a hydrogen atom, methyl, ethyl, formyl or acetyl group;
$R_2$ represents a methyl, amino, methylamino, N,N-dimethylamino, ethylamino, N,N-diethylamino, N-methyl-N-ethylamino, 1-pyrrolidinyl, (6-chloro-3-pyridyl)methylamino or N-methyl-N-(6-chloro-3-pyridyl)methylamino group; and
X represents a nitrogen atom or CH group;
Y represents a cyano, nitro or trifluoroacetyl group;
m represents an integer of 0 or 1),
as well as, a method for controlling ectoparasites of animals.

The present compounds are known as active ingredients for pesticides, and described in, for example, US-A-5,532,365, US-A-4,742,060, US-A-4,849,432, US-A-5,034,404, US-A-5,750,548, US-A-5,304,566 and EP-A-0 428

941. The present compound is comprised from 0.1 to 20% by weight, preferably from 1 to 15 % by weight, and more preferably from 5 to 10% by weight in the present agent.

As the compound given in the formula (1), for example, 1-(tetrahydrofuran-3-yl)methyl-3-methyl-2-nitroguanidine or 1-(tetrahydrofuran-2-yl)methyl-3-methyl-2-nitroguanidine is suitable.

As the glycol or glycol monoalkyl ether, for example, ethylene glycol, diethylene glycol, triethylene glycol, propylene glycol, dipropylene glycol, tripropylene glycol, hexamethylene glycol, 1,3-butanediol, 3-methyl-1,3-butanediol, 2-methyl-1,3-propanediol, ethylene glycol monomethyl ether, ethylene glycol monoethyl ether, ethylene glycol monobutyl ether, diethylene glycol monomethyl ether, diethylene glycol monoethyl ether, propylene glycol monomethyl ether, propylene glycol monoethyl ether, propylene glycol monotertiarybutyl ether, dipropylene glycol monomethyl ether or 3-methoxy-3-methyl-1-butanol is suitable. The present agent comprises the glycol from 10 to 95% by weight, preferably from 30 to 90% by weight, and more preferably from 50 to 80% by weight.

More specifically, for the present compound and the glycol in the present agent, the following are examples of suitable combinations:

1-(tetrahydrofuran-3-yl)methyl-3-methyl-2-nitroguanidine and ethylene glycol,
1-(tetrahydrofuran-3-yl)methyl-3-methyl-2-nitroguanidine and diethylene glycol,
1-(tetrahydrofuran-3-yl)methyl-3-methyl-2-nitroguanidine and triethylene glycol,
1-(tetrahydrofuran-3-yl)methyl-3-methyl-2-nitroguanidine and propylene glycol,
1-(tetrahydrofuran-3-yl)methyl-3-methyl-2-nitroguanidine and dipropylene glycol,
1-(tetrahydrofuran-3-yl)methyl-3-methyl-2-nitroguanidine and tripropylene glycol,
1-(tetrahydrofuran-3-yl)methyl-3-methyl-2-nitroguanidine and hexamethylene glycol,
1-(tetrahydrofuran-3-yl)methyl-3-methyl-2-nitroguanidine and 1,3-butanediol,
1-(tetrahydrofuran-3-yl)methyl-3-methyl-2-nitroguanidine and 3-methyl-1,3-butanediol,
1-(tetrahydrofuran-3-yl)methyl-3-methyl-2-nitroguanidine and 2-methyl-1,3-propanediol,
1-(tetrahydrofuran-3-yl)methyl-3-methyl-2-nitroguanidine and ethylene glycol monomethyl ether,
1-(tetrahydrofuran-3-yl)methyl-3-methyl-2-nitroguanidine and ethylene glycol monoethyl ether,
1-(tetrahydrofuran-3-yl)methyl-3-methyl-2-nitroguanidine and ethylene glycol monobutyl ether,
1-(tetrahydrofuran-3-yl)methyl-3-methyl-2-nitroguanidine and propylene glycol monomethyl ether,
1-(tetrahydrofuran-3-yl)methyl-3-methyl-2-nitroguanidine and propylene glycol monoethyl ether,
1-(tetrahydrofuran-3-yl)methyl-3-methyl-2-nitroguanidine and propylene glycol monotertiarybutyl ether,
1-(tetrahydrofuran-3-yl)methyl-3-methyl-2-nitroguanidine and 3-methoxy-3-methyl-1-butanol, and so on.

The present agent is a liquid formulation comprising 10 to 95% by weight of a glycol or glycol monoalkyl ether and 0.1 to 20% by weight of the present compound, and may comprise, optionally, for example, liquid carriers such as $C_{1-4}$ alcohols (e.g. methanol, ethanol, isopropyl alcohol, tert-butyl alcohol); propylene carbonate, N-methyl-2-pyrrolidone and water. Said liquid carrier is preferably the solvent that is miscible with a glycol or glycol monoalkyl ether and the amount of the solvent is usually 3 to 85% by weight or less, preferably 3 to 70% by weight or less in case that the solvent is utilized. The present agent may further comprise auxiliaries. Examples of the auxiliaries include anti-oxidants such as BHT and BHA; emulsifiers such as sorbitan monooleate, sorbitan monolaurate, caprylic acid monoglyceride, capric acid monoglyceride, isostearic acid monoglyceride and propylene glycol monocaprylate; and oxyacid esters such as triethyl citrate.

The present agent may also comprise, optionally, active ingredients other than the present compound.

The active ingredients are, for example, pyrethroid compounds such as permethrin, phenothrin, allethrin, pyrethrin, cyphenothrin, cyfluthrin, fenvalerate, fenpropathrin and transfluthrin; organophosphorus compounds such as dichlorvos, tetrachlorvinphos, fenthion, chlorpyrifos and diazinon; carbamate compounds such as propoxur, carbaryl, metoxadiazone and fenobucarb; chitin-synthesis inhibiting substances such as lufenuron, chlorfluazuron, hexaflumuron, cyromazine and 1-(2,6-difluorobenzoyl)-3-[2-fluoro-4-(1,1,2, 3, 3, 3-hexafluoropropoxy)phenyl]urea; juvenile hormone analogues such as methoprene, hydroprene and fenoxycarb; N-phenylpyrazole compounds; endoparasite controlling substances for animals such as milbemycin, abamectin and avermectin; pest repelling compounds such as N,N-diethyl-m-toluamide (DEET), limonene, linalool, citronellal, menthol, menthone, hinokitiol, geraniol, eucalyptol, indoxacarb, carane-3,4-diol, orange oil, citronella oil, lemon oil, lemongrass oil, cinnamon oil, peppermint oil, spearmint oil and hyssop oil; or synergists such as piperonyl butoxide (PBO), octachlorodipropyl ether (S-421), N-(2-ethylhexyl) bicyclo[2.2.1]hept-5-ene-2,3-dicarboximide, isobornyl thiocyanatoacetate (IBTA) and N-(2-ethylhexyl)-1-isopropyl-4-methylbicyclo[2.2.2]oct-5-ene-2,3-dicarboximide are suitable.

The present agent usually controls ectoparasites for animals efficiently by employing onto animals by regional application methods such as spot-on application and pour-on application.

The spot-on application is a well-known method for controlling ectoparasites by dropping a liquid agent onto a skin at the back of blade bone of animal body.

The pour-on application is also a well-known method for controlling ectoparasites by pouring a liquid agent along the back line of animal body and having the liquid agent spread on the surface of the animal body.

The dosage of the present agent is usually about 0.01 to 10mL and the dosage of the present compound is usually about 0.1 to 300mg per 1kg of animal weight.

For the present invention, and as the pests that are effectively controlled, suitable ectoparasites of farm animals such as cows and sheep, as well as pets such as dogs and cats, include, for example, Diptera such as *Musca hervei, Musca bezzii, Haematobia irritans*, *Simulium iwatens*, *Culicoides oxystoma*, *Tabanus chrysurus*, *Culex pipiens* (common mosquito) and *Aedes albopictus*; Anoplura (lice) such as *Haemaropinus eurysternus* and *Damalinia ovis;* and Siphonaptera such as *Ctenocephalides felis* (cat flea), *Ctenophalides canis* (dog flea) and *Xenopsylla cheopis.*

Furthermore, the objective animals that the present invention is employed upon, include the farm animals and pets mentioned above, and others, for example, Rodentia such as mice, rats, hamsters and squirrels; Lagomorpha such as rabbits; Carnivora such as ferrets; birds such as ducks, chickens, doves; and the like.

Examples

The present invention is explained with the examples in detail below.

Formulation example 1

Five parts by weight of 1-(tetrahydrofuran-3-yl)methyl-3-methyl-2-nitroguanidine (hereinafter, Compound A), 50 parts by weight of hexamethylene glycol and 45 parts by weight of isopropanol are mixed to obtain a formulation.

Formulation example 2

Five parts by weight of Compound A, 50 parts by weight of propylene glycol monomethyl ether and 45 parts by weight of dipropylene glycol are mixed to obtain a formulation.

Formulation example 3

Five parts by weight of Compound A, 0.5 parts by weight of pyriproxyfen, 50 parts by weight of propylene glycol monomethyl ether and 44.5 parts by weight of isopropanol are mixed to obtain a formulation.

Formulation example 4

Five parts by weight of Compound A, 85 parts by weight of propylene glycol monomethyl ether and 10 parts by weight of d-limonen are mixed to obtain a formulation.

Test example 1

Formulations 1 through 7 were obtained by dissolving 5 parts by weight of Compound A into a previously prepared mixed solution of tripropylene glycol (hereinafter, TPG) and isopropanol (hereinafter, IPA) wherein the parts by weight of each preparation of 7 possibilities is 0:95, 10:85, 30:65, 50:45, 70:25, 80:15 and 95:0.

One fiftieths milliliters (0.02mL) was dropped onto the neck area epiderrnis of a mouse (weight: about 30g). Said mouse was hold and fastened with a metal net, and placed into a 900mL glass canister. Twenty adult cat fleas were deposited into said canister so the mouse is infested. One day after infestation, the mortality was examined. Each was repeated thrice. The results are given in table 1.

Table 1

| Test # | Formulation | TPG/IPA (wt/wt) | Compound A/ TPG (wt/wt) | Mortality of Cat fleas (%) |
|---|---|---|---|---|
| 1-1(Comparative) | 1 | 0/95 | 5/0 | 38.3 |
| 1-2 | 2 | 10/85 | 5/10 | 55.0 |
| 1-3 | 3 | 30/65 | 5/30 | 75.0 |
| 1-4 | 4 | 50/45 | 5/50 | 80.0 |
| 1-5 | 5 | 70/25 | 5/70 | 93.3 |

Table 1 (continued)

| Test # | Formulation | TPG/IPA (wt/wt) | Compound A/ TPG (wt/wt) | Mortality of Cat fleas (%) |
|---|---|---|---|---|
| 1-6 | 6 | 80/15 | 5/80 | 75.0 |
| 1-7 | 7 | 95/0 | 5/95 | 60.0 |

Test example 2

Ten parts by weight of Compound A was mixed with 90 parts by weight of diethylene glycol monoethyl ether and dissolved to afford a spot-on formulation.

Thirty cat flea adults were deposited on a cat (weight: 3.4kg) and an elizabethan collar was put on the neck of the cat the day before application of the above formulation. Two fifths mililiters (0.4mL) of the above spot-on formulation was spread onto the skin at the back of blade bone of the cat. Then, one day and three days after application, the number of infested fleas was counted by using a comb for gathering fleas. All the counted fleas were re-deposited on the cat after counting.

In addition, as a blank, cat fleas were deposited on a cat without application of the formulation by the same method as above, the number of the cat fleas was observed and controlling ratio was calculated by the following formula:

$$\text{Controlling ratio (\%)} = (C\text{-}T) \div C \times 100$$

C : Infesting ratio of fleas in the case without application
T : Infesting ratio of fleas in the case with application of the formulation

Each was repeated twice and the result are given in table 2.

Table 2

| | | One day after application | Three days after application |
|---|---|---|---|
| treated with spot-on formulation | Infesting ratio | 3.3 | 1.7 |
| | Controlling ratio | 95.6 | 97.5 |
| blank (no treatment) | Infesting ratio | 75.0 | 66.7 |

**Claims**

**1.** An ectoparasite-controlling agent for animals which is a liquid formulation comprising 0.1 to 20% by weight of the neonicotinoid compound given in the following formula (1) and 10 to 95% of a glycol or glycol monoalkyl ether:

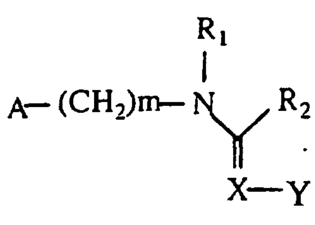

(1)

wherein, A represents a tetrahydrofuran-2-yl, tetrahydrofuran-3-yl, or 5-methyltetrahydrofuran-3-yl group;
$R_1$ represents a hydrogen atom, methyl, ethyl, formyl or acetyl group; $R_2$ represents a methyl, amino, methylamino, N,N-dimethylamino, ethylamino, N,N-diethylamino, N-methyl-N-ethylamino, 1-pyrrolidinyl, (6-chloro-3-pyridinyl) methylamino or N-methyl-N-(6-chloro-3-pyridinyl)methylamino group;
X represents a nitrogen atom or CH group;
Y represents a cyano, nitro or trifluoroacetyl group;

and m represents an integer of 0 or 1.

2. An ectoparasite-controlling agent according to claim 1, wherein the glycol or glycol monoalkyl ether is at least one selected from ethylene glycol, diethylene glycol, triethylene glycol, propylene glycol, dipropylene glycol, tripropylene glycol, hexamethylene glycol, 1,3-butanediol, 3-methyl-1,2-butanediol, 2-methyl-1,3-propanediol, ethylene glycol monomethyl ether, ethylene glycol monoethyl ether, ethylene glycol monobutyl ether, diethylene glycol monomethyl ether, diethylene glycol monoethyl ether, propylene glycol monomethyl ether, propylene glycol monoethyl ether, propylene glycol monotertiarybutyl ether, dipropylene glycol monomethyl ether and 3-methoxy-3-methyl-1-butanol.

3. An ectoparasite-controlling agent according to claim 1 or 2, which further comprises a solvent that is miscible with the glycol or glycol monoalkyl ether.

4. An ectoparasite-controlling agent according to claim 1 or 2, which further comprises a solvent selected from $C_{1-4}$ alcohols, propylene carbonate, N-methyl-2-pyrrolidone and water.

5. An ectoparasite-controlling agent according to claim 1, wherein the substituent A is a tetrahydrofuran-2-yl or tetrahydrofuran-3-yl group.

6. An ectoparasite-controlling agent according to claim 1, wherein the neonicotinoid compound is 1-(tetrahydrofuran-3-yl)methyl-3-methyl-2-nitroguanidine.

7. A use of the ectoparasite-controlling agent of claim 1 for the manufacture of a medicament for spot-on or pour-on application for animals.

**Patentansprüche**

1. Ektoparasiten bekämpfendes Mittel für Tiere, bei welchem es sich um eine flüssige Formulierung handelt, die 0,1 bis 20 Gew.-% der Neonikotinoidverbindung der folgenden Formel (1) und 10 bis 95 Gew.-% eines Glycols oder eines Glycolmonoalkylethers umfasst:

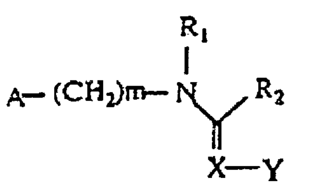

(1)

wobei

A eine Tetrahydrofuran-2-yl-, Tetrahydrofuran-3-yl- oder 5-Methyltetrahydrofuran-3-yl-Gruppe darstellt;
$R_1$ ein Wasserstoffatom, eine Methyl-, Ethyl-, Formyl- oder Acetylgruppe darstellt;
$R_2$ eine Methyl-, Amino-, Methylamino-, N,N-Dimethylamino-, Ethylamino-, N,N-Diethylamino-, N-Methyl-N-ethylamino-, 1-Pyrrolidinyl-, (6-Chlor-3-pyridinyl)methylamino- oder N-Methyl-N-(6-chlor-3-pyridinyl)methylaminogruppe darstellt;
X ein Stickstoffatom oder eine CH-Gruppe darstellt;
Y eine Cyano-, Nitro- oder Trifluoracetylgruppe darstellt; und
m eine ganze Zahl von 0 oder 1 darstellt.

2. Ektoparasiten bekämpfendes Mittel nach Anspruch 1, wobei das Glycol oder der Glycolmonoalkylether mindestens ein aus Ethylenglycol, Diethylenglycol, Triethylenglycol, Propylenglycol, Dipropylenglycol, Tripropylenglycol, Hexamethylenglycol, 1,3-Butandiol, 3-Methyl-1,2-butandiol, 2-Methyl-1,3-propandiol, Ethylenglycolmonomethylether, . Ethylenglycolmonoethylether, Ethylenglycolmonobutylether, Diethylenglycolmonomethylether, Diethylenglycolmonoethylether, Propylenglycolmonomethylether, Propylenglycolmonoethylether, Propylenglycolmono-tert.-butylether, Dipropylenglycolmonomethylether und 3-Methoxy-3-methyl-1-butanol ausgewähltes(r) ist.

3. Ektoparasiten bekämpfendes Mittel nach Anspruch 1 oder 2, weiterhin umfassend ein Lösungsmittel, das mit dem Glycol oder dem Glycolmonoalkylether mischbar ist.

4. Ektoparasiten bekämpfendes Mittel nach Anspruch 1 oder 2, weiterhin umfassend ein Lösungsmittel, ausgewählt aus $C_{1-4}$-Alkoholen, Propylencarbonat, N-Methyl-2-pyrrolidon und Wasser.

5. Ektoparasiten bekämpfendes Mittel nach Anspruch 1, wobei Substituent A eine Tetrahydrofuran-2-yl- oder Tetrahydrofuran-3-yl-Gruppe ist.

6. Ektoparasiten bekämpfendes Mittel nach Anspruch 1, wobei die Neonikotinoidverbindung 1-(Tetrahydrofuran-3-yl) methyl-3-methyl-2-nitroguanidin ist.

7. Verwendung des Ektoparasiten bekämpfenden Mittels nach Anspruch 1 zur Herstellung eines Medikaments für die punktuelle oder flächige Verabreichung an Tiere.

## Revendications

1. Agent de lutte contre les ectoparasites chez les animaux, qui est une formulation liquide comprenant 0,1 à 20 % en masse du composé néonicotinoïde représenté dans la figure (1) suivante et 10 à 95 % d'un glycol ou d'un éther de monoalkyle de glycol :

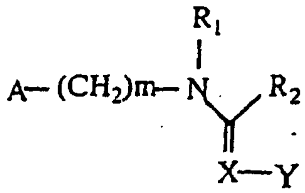

(1)

où A représente un groupe tétrahydrofuran-2-yle, tétrahydrofuran-3-yle ou 5-méthyltétrahydrofuran-3-yle ; $R_1$ représente un atome d'hydrogène, un groupe méthyle, éthyle, formyle ou acétyle ; $R_2$ représente un groupe méthyle, amino, méthylamino, N,N-diméthylamino, éthylamino, N,N-diéthylamino, N-méthyl-N-éthylamino, 1-pyrrolidinyle, (6-chloro-3-pyridinyl)méthylamino ou N-méthyl-N-(6-chloro-3-pyridinyl)méthylamino ; X représente un atome d'azote ou un groupe CH ; Y représente un groupe cyano, nitro ou trifluoroacétyle ; et m représente un entier égal à 0 ou 1.

2. Agent de lutte contre les ectoparasites selon la revendication 1, dans lequel le glycol ou l'éther de monoalkyle de glycol est au moins un élément choisi parmi l'éthylèneglycol, le diéthylèneglycol, le triéthylèneglycol, le propylèneglycol, le dipropylèneglycol, le tripropylèneglycol, l'hexaméthylèneglycol, le 1,3-butanediol, le 3-méthyl-1,2-butanediol, le 2-méthyl-1,3-propanediol, l'éther de monométhyle de l'éthylèneglycol, l'éther de monoéthyle de l'éthylèneglycol, l'éther de monobutyle de l'éthylèneglycol, l'éther de monométhyle du diéthylèneglycol, l'éther de monoéthyle du diéthylèneglycol, l'éther de monométhyle du propylèneglycol, l'éther de monoéthyle du propylèneglycol, l'éther de mono-tertio-butyle du propylèneglycol, l'éther de monométhyle du dipropylèneglycol et le 3-méthoxy-3-méthyl-1-butanol.

3. Agent de lutte contre les ectoparasites selon la revendication 1 ou 2, qui comprend en outre un solvant miscible avec le glycol ou l'éther de monoalkyle de glycol.

4. Agent de lutte contre les ectoparasites selon la revendication 1 ou 2, qui comprend en outre un solvant choisi parmi les alcools en $C_{1-4}$, le carbonate de propylène, la N-méthyl-2-pyrrolidone et l'eau.

5. Agent de lutte contre les ectoparasites selon la revendication 1, où le substituant A est un groupe tétrahydrofuran-2-yle ou tétrahydrofuran-3-yle.

6. Agent de lutte contre les ectoparasites selon la revendication 1, où le composé néonicotinoïde est la 1-(tétrahydrofuran-3-yl)méthyl-3-méthyl-2-nitroguanidine.

7. Utilisation de l'agent de lutte contre les ectoparasites selon la revendication 1, pour la fabrication d'un médicament pour une application animale destiné à être déposé par points ou déversé.